(19) Europäisches Patentamt
European Patent Office
Office européen des brevets

(11) **EP 0 597 995 B1**

(12) **EUROPÄISCHE PATENTSCHRIFT**

(45) Veröffentlichungstag und Bekanntmachung des Hinweises auf die Patenterteilung:
**04.06.1997 Patentblatt 1997/23**

(51) Int. Cl.$^6$: **A61N 1/05**

(21) Anmeldenummer: 92917166.8

(86) Internationale Anmeldenummer:
**PCT/DE92/00658**

(22) Anmeldetag: **06.08.1992**

(87) Internationale Veröffentlichungsnummer:
**WO 93/02739 (18.02.1993 Gazette 1993/05)**

(54) **STIMULATIONSELEKTRODE**

HEART-STIMULATION ELECTRODE

ELECTRODE DE STIMULATION

(84) Benannte Vertragsstaaten:
**DE FR GB IT NL SE**

(30) Priorität: **06.08.1991 DE 4126362**
**05.03.1992 DE 4207368**

(43) Veröffentlichungstag der Anmeldung:
**25.05.1994 Patentblatt 1994/21**

(73) Patentinhaber: **BIOTRONIK Mess- und Therapiegeräte GmbH & Co Ingenieurbüro Berlin D-12359 Berlin (DE)**

(72) Erfinder:
• **BOLZ, Armin D-8520 Erlangen (DE)**
• **SCHALDACH, Max D-8520 Erlangen (DE)**

(74) Vertreter: **Christiansen, Henning, Dipl.-Ing. et al Patentanwalt Pacelliallee 43/45 14195 Berlin (DE)**

(56) Entgegenhaltungen:
**EP-A- 0 085 743        EP-A- 0 115 778**
**US-A- 4 280 514**

• **BIOMEDIZINISCHE TECHNIK Bd. 34, Nr. 7/8, August 1989, BERLIN, DE Seiten 185 - 190 SCHALDACH 'Titannitrid-Herzschrittmacher-Elektroden'**

## Beschreibung

Die Erfindung betrifft eine Stimulationselektrode der im Oberbegriff des Anspruchs 1 angegebenen Art.

Aus elektrischer Sicht läßt sich die Phasengrenze Zwischen einem Festkörper, also zwischen der Stimulationselektrode eines Herzschrittmachers und einem Elektrolyten vereinfacht als Parallelschaltung der Phasengrenz- d.h. der Helmholtzkapazität $C_H$ und des Faradaywiderstandes $R_F$ beschreiben, dem der Leitungswiderstand $R_L$ in Serie geschaltet ist. Damit ist die Impedanz des Elektrodensystems $Z_{DL}$ von der Frequenz $\omega$ der angelegten Spannung nach folgender Gleichung abhängig:

$$Z_{DL} = R_L + (\frac{1}{R_F^2} + \omega^2 C_H^2)^{-1/2} \qquad (1)$$

Für die Erregung des Herzmuskels ist eine bestimmte Ladung Q erforderlich, die sich aus dem Integral des Stimulationsstromes I(t) über die Pulsbreite T berechnen läßt. Daraus folgt, daß man durch eine Minimierung der Impedanz des Elektrodensystems $Z_{DL}$ die für die Erregung des Herzens erforderliche Energiemenge

$$E \approx \int_0^T U(t) I(t) dt$$

ebenfalls minimieren kann. Da der Leitungswiderstand $R_L$ konstant ist, der Faradaywiderstand $R_F$ mit folgender Gleichung

$$R_F = \frac{R_o}{A} \qquad (2)$$

definiert wird, wobei $R_o$ ein konstanter Überleitungswiderstand und A die aktive Oberfläche ist, und die Helmholtzkapazität $C_H$ wie folgt definiert wird:

$$C_H = \varepsilon \cdot \varepsilon_o \frac{A}{d}, \qquad (3)$$

wobei $\varepsilon$ die Dielektrizitätskonstante der angelagerten Wasserdipole, $\varepsilon_o$ die Dielektrizitätskonstante des Vakuums und d die Dicke der Helmholtzschicht ist, führt die Vergrößerung der aktiven Oberfläche der Elektrode gemäß (3) zur Vergrößerung der Helmholtzkapazität $C_H$ und gemäß (2) zur Verminderung des Faradaywiderstandes $R_F$ Beide haben dann gemäß (1) eine Verringerung der Impedanz $Z_{DL}$ und der erforderlichen Energiemenge E zur Folge. Die aktive Oberfläche A ist dabei insbesondere durch die Vergrößerung der Elektrode und/oder durch eine Strukturierung der Elektrodenoberfläche veränderbar.

Aus EP-A-0 117 972, EP-A-0 116 280, EP-A-0 115 778 und Schaldach, M. et al.: "Titannitrid-Herzschrittmacher-Elektroden", Biomedizinische Technik, 34 (1989), Nr. 7, S.185 sind bereits Stimulationselektroden bekannt, deren elektrochemisch aktiven Oberflächen mittels einer porösen Schicht aus einem Carbid, Nitrid oder Carbonitrid wenigstens eines der Metalle Titan, Vanadium, Zirkon, Niob, Molybdän, Hafnium, Tantal oder Wolfram vergrößert sind.

Aus EP-A-0 085 743 ist eine ähnliche Elektrode bekannt, deren Kopf aus miteinander versinterten Ir- oder Ir-Legierungs-Kügelchen besteht.

Nachteilig bei diesen bekannten porösen Elektrodenbeschichtungen ist aber, daß die Gesamtkapazität der implantierten Elektroden sich mit der Zeit langsam verringert und zu einer entsprechenden Erhöhung der erforderlichen Energiemenge führt. Damit muß die Stimulationsspannung relativ hoch gewählt werden, um mit der Impulsenergie die Reizschwelle der Patienten auch langfristig Zu übertreffen. Zur Abgabe der erhöhten Energie ist aber eine Erhöhung der Spannung der Impulse notwendig, woraus wiederum eine Vergrößerung der Energiequellen - und damit eine Vergrößerung des Gehäuses - bei implantierbaren Systemen - resultiert. Mit der Erhöhung der Impulsenergie erhöht sich auch die Polarisationsspannung, so daß auch die üblicherweise verwendeten Gegenimpulse bei zur Vermeidung der Auswirkungen der Polarisationsspannung auf den Eingangsverstärker des Schrittmachers nach erfolgter Stimulation entsprechend vergrößert werden müssen.

Der Erfindung liegt die Aufgabe zugrunde, eine Stimulationselektrode der eingangs genannten Gattung derart zu verbessern, daß zum einen die erforderliche Energie zur Stimulation auch langfristig niedrig bleiben kann und daß zum anderen eine sichere Effektivitätserkennung mit einfachen Maßnahmen gewährleistet ist.

Diese Aufgabe wird mit den kennzeichnenden Merkmalen des Anspruchs 1 gelöst.

Die Erfindung beruht auf der Erkenntnis, daß die Werkstoffe der bekannten Elektroden und insbesondere Titan, Vanadium, Zirkon und Niob zu teilweise extremer Oxidation neigen und daß diese hohe Oxidationsneigung bei Kontakt zu wässrigen Elektrolyten dazu führt, daß sich an der Elektrodenoberfläche eine dünne, isolierende bzw. halbleitende Oxidschicht bildet, die eine der Helmholtzkapazität $C_H$ in Serie geschaltete Kapazität $C_{ox}$ darstellt und so zur langsamen Verringerung der Gesamtkapazität und damit zur entsprechenden Erhöhung der jeweils erforderlichen Stimulationsenergie führt. Bei anodischer Polung werden $OH^-$-Ionen in den Festkörper gezogen und führen dort zur Vergrößerung der Oxidschichtdicke führen. Dies hat eine weitere Verringerung der Phasengrenzkapazität und damit eine weitere Erhöhung der Elektrodenimpedanz zur Folge. Die anodischen Pulse, die bei der Effektivitätserkennung bei dem üblichen Ladungsintegrationsverfahren als aktive Gegenpulse erforderlich sind, bewirken daher, daß die Effektivitätserkennung mit den bekannten Elektroden nicht oder nur bei einer erhöhten Energiemenge durchführbar ist.

Eine anodische Polung tritt aber nicht nur bei aktiven Gegenimpulsen zur Effektivitäterkennung auf, sondern auch bei anodisch gepolter Elektrode in multipolaren Schrittmachersystemen oder bei der Impedanzmessung im Herzen. Sie kann darüber hinaus auch durch Überschwinger der Stimulationsimpulse hervorgerufen werden.

Damit ist den herkömmlichen beschichteten porösen Elektroden wegen ihrer großen relativen Oberfläche zunächst eine grundsätzlich eine Stimulation mit gutem Erfolg bei niedriger Energie möglich. Es wurde nun erkannt, daß durch die Oxidationsneigung die Helmholtzkapazität verkleinert wird, was zu einer Erhöhung der Elektrodenimpedanz führt. Die damit hervorgerufene Beeinflussung der Elektrodeneigenschaften im Laufe der Implantationszeit ist deshalb so schwerwiegend, weil die Verschlechterung der Elektrodeneigenschaften Auswirkungen hat, welche ihrerseits dazu beitragen, daß die Stimulationseigenschaften zusätzlich ungünstig beeinflußt werden. So ist bei einer sich verschlechternden Elektrode eine höhere Impulsenergie notwendig, so daß zur Effektivitätserkennung auch ein Gegenimpuls mit größerer Energie notwendig ist, der seinerseits wieder zur Verschlechterung der Elektrodeneigenschaften beiträgt. Da die Impulsenergie und die zur Effektivitätserkennung notwendigen Gegenimpulse auf Werte eingestellt sind, welche über die gesamte Implantationsdauer des Schrittmachers Gültigkeit haben müssen, beruht die Verschlechterung der Betriebsbedingungen, im Endeffekt im wesentlichen auf Maßnahmen, welche den verschlechterten Betriebsbedingungen eigentlich entgegenwirken sollen.

Die langzeitstabile, bioverträgliche Oberflächenbeschichtung der erfindungsgemäßen Stimulationselektrode besteht aus einem Material dessen Oxidationsneigung sehr gering ist, wobei sie vorzugsweise unter Verwendung eines inerten Materials, also eines Nitrides, Carbides, Carbonitrides oder aber eines reinen Elementes bzw. bestimmter Legierungen aus der Gruppe Gold, Platin, Iridium oder Kohlenstoff vakuumtechnisch auf die Elektrode aufgetragen wird. Wegen der fraktalen räumlichen Geometrie einer derart aufgetragenen Oberflächenschicht ist deren aktive Oberfläche sehr groß, so daß die zur Stimulation erforderliche Energiemenge gering gehalten werden kann.

Das Nachpotential einer Stimulationselektrode aus Titan, die eine mittels der reaktiven Kathodenzerstäubung gesputterte Iridiumschicht aufweist, ist bis um das Sechsfache (von ca. 600 auf ca. 100 mV) kleiner als das Nachpotential einer blanken Stimulationselektrode aus Titan. Wegen dieser signifikanten Verringerung des Nachpotentials ist die Erkennung des intrakardialen EKGs nicht nur auf herkömmliche Weise mit einem Verstärker und einer Triggereinrichtung möglich, sondern es kann eine funktionsfähige Effektivitätserkennung angewandt werden, die ohne Gegenimpuls auskommt.

Durch die Verringerung der erforderlichen Stimulationsenergie über die Lebensdauer des Implantats kann auf sonst erforderliche Reserven verzichtet und in vorteilhafter Weise die Betriebszeit des Implantates entscheidend vergrößert bzw. die Gehäusegröße entscheidend verkleinert werden.

Zur erfolgreichen Stimulation ist eine bestimmte Ladung Q erforderlich. Der dazu notwendige Strom lädt auch die Helmholtzkapazität $C_H$ auf, weshalb nach dem Stimulus eine Spannung, das sogenannte Nachpotential, über dem Kondensator meßbar ist. Da bei konstanter Ladung die an einem Kondensator abfallende Spannung invers proportional zur Kapazität ist, wird auch das Nachpotential durch eine hohe Helmholtzkapazität $C_H$, die durch die große aktive Oberfläche der erfindungsgemäßen Stimulationselektrode erzielt wird, herabgesetzt und seine zeitliche Änderung verringert. Da die inerte Oberflächenschicht der erfindungsgemäßen Stimulationselektrode keine bzw. nur eine sehr geringe Oxidationsneigung aufweist, kann - falls trotzdem unter bestimmten Bedingungen gewünscht - die Elektrode anodisch betrieben werden, ohne daß sich eine Oxidschicht bildet und/oder deren Schichtdicke d sich vergrößert, so daß die Helmholtzkapazität $C_H$ stets auf einem hohen Wert gehalten werden kann, wobei das durch die Elektrode verursachte Nachpotential wie erwünscht gering gehalten wird und somit für die Optimierung des Stimulationsverhaltens durch eine sichere Effektivitätserkennung gesorgt wird.

Die Eigenschaften der erfindungsgemäßen Elektrode werden durch die fraktale Geometrie gegenüber Elektroden nach dem Stand der Technik wesentlich verbessert, da durch die fraktalartige "blumenkohlartige Oberfläche" poröse Strukturen geschaffen werden, welche eine Feinstruktur mit in Bezug auf eine die äußere Geometrie der Elektrode umhüllenden Fläche eine sehr große Oberflächenzunahme aufweisen. Durch die geometrischen Bereiche, welche im Zusammenhang mit der fraktalen Geometrie die gröbere Struktur aufweisen, werden andererseits Bezirke geschaffen, die für eine ausreichende mechanische Festigkeit sorgen und als Träger für die Bereiche mit feinerer geometrischer Struktur dienen. Es ist also ersichtlich, daß die aktive BeSchichtung der Elektrode eine geometrische Struktur aufweist, die sich zu ihrer Oberfläche hin zunehmend verfeinert. Die Größe der Poren nimmt also mit zunehmender Nähe zur Oberfläche ab. Vergleichbar ist eine derartige Struktur mit einem Adersystem, welches in seinen peripheren Bereichen eine Feinstruktur aufweist, die in ein zunehmend gröber strukturiertes Hauptadersystem münden.

Da das Frequenzspektrum der intrakardialen Signale eine Bandbreite bis etwa 50 Hz mit einem Maximum bei etwa 1 bis 5 Hz besitzt, läßt sich auch mit der Maximierung der Helmholtzkapazität $C_H$ das Übertragungsverhalten, vor allem das der erheblichen niederfrequenten Anteile des Frequenspektrums optimieren.

Weiterhin vorteilhaft bei der erfindungsgemäßen Stimulationselektrode ist, daß die Signalamplituden bei der Detektion vergrößert werden, da die detektierte Spannung in allen Frequenzbereichen von der Gesamtimpedanz des Elektrodensystems $Z_S$ und der Phasengrenzimpedanz nach folgender Gleichung beruht ($U_{EKG}$ entspricht dabei der im Herzen tatsächlich vorliegenden Spannung des intrakardialen EKGs):

$$U_{det} \approx U_{EKG} \left( \frac{Z_S - Z_{DL}}{Z_S} \right) \qquad (4)$$

und durch die Maximierung der Helmholtzkapazität $C_H$ die Impedanz des Elektrodensystems $Z_{DL}$ minimiert wird.

Obwohl die Größe der aktiven Oberfläche durch eine einfache Vergrößerung der Elektrode zu verändern wäre, hat es sich herausgestellt, daß es vorteilhafter ist, die aktive Oberfläche im Verhältnis zur sich aus der geometrischen Form der Elektrode ergebenden Oberfläche zu maximieren, da eine lineare Vergrößerung auch nur eine näherungsweise oberflächenproportionale Erhöhung der zur Reizung erforderlichen Ladung Q zur Folge hat und daher keine Lösung darstellt. Diese Beobachtung erklärt sich durch den unterschiedlichen Einflußbereich der Stimulationselektroden; in einer verfeinerten Sprechweise müßte eigentlich von einer konstanten, für die Herzmuskelerregung erforderlichen Ladungsdichte gesprochen werden.

Die erfindungsgemäßen Oberflächenbeschichtungen aus den genannten Werkstoffen, und insbesondere aus Iridiumnitrid IrN, die mit Hilfe moderner Vakuumbeschichtungsverfahren wie Sputtern oder Ionenplattieren auf herkömmliche Elektroden aufgebracht werden, sorgen aufgrund ihrer fraktalen Geometrie für Oberflächenvergrößerungen um einen Faktor 1000 und mehr. Bei einer fraktalen Geometrie wird eine Anzahl eines Elements wiederholt aber verkleinert auf größeren Elementen mit annähernd gleicher Form aufgefunden. Eine derartige Formgebung läßt sich - mindestens angenähert - mit Verfahren der Dünnschichttechnologie bei entsprechender Einstellung der Verfahrensparameter erzielen. Die erfindungsgemäße Elektrode weist auch langfristig überraschend niedrige Stimulationsschwellwerte auf.

Durch die Möglichkeit der anodischen Betriebsweise läßt sich die Elektrode in günstiger Weise auch für Betriebsweisen einsetzen, bei denen diese Polarität funktionsnotwendig ist, wie beispielsweise bei bi- oder multipolaren Elektroden oder intrakardialer Impedanzmessung.

Die erfindungsgemäße Elektrode ist in bevorzugter Weise auch für die Neurostimulation und generell für solche Stimulationszwecke geeignet, bei denen es nicht auf hohe Feldstärken, sondern auf eine geringe Impedanz und damit auf große lokale Ladungs- bzw. Stromdichte benachbart zu dem zu stimulierenden Organ bzw. den betreffenden Nervenleitbahnen ankommt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Ausführungsbeispiel einer erfindungsgemäßen Stimulationselektrode in schematischer Darstellung in Seitenansicht,

Figur 2 eine vergrößerte Darstellung des Details II der Figur 1 im Schnitt,

Figur 3 ein Diagramm zum Vergleich der Impedanz des Ausführungsbeispiels der erfindungsgemäßen Elektrode mit aus dem Stand der Technik bekannten entsprechenden Elektroden gleicher geometrischer Abmessung,

Figur 4 eine Darstellung zur fraktalen Oberflächengeometrie der erfindungsgemäßen Elektrode sowie

Figur 5 ein Ausschnitt der Oberfläche der erfindungsgemäßen Elektrode in vergrößerter Darstellung.

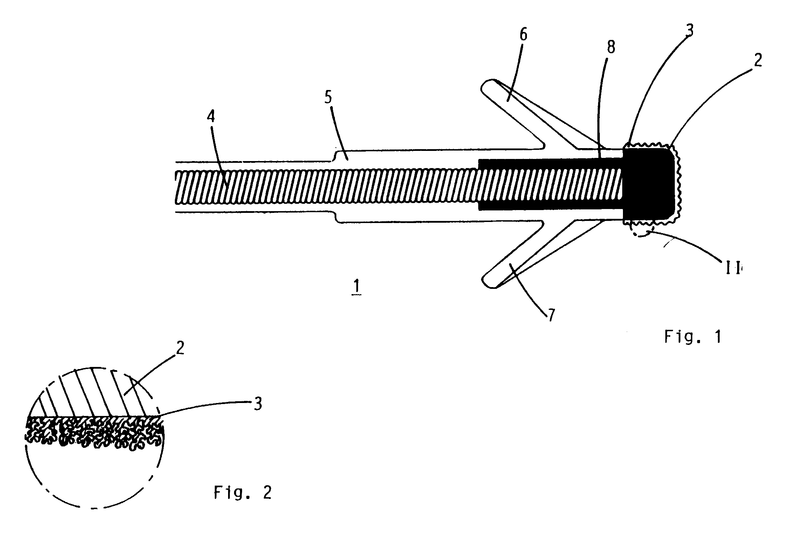

Bei der in Figur 1 in schematischer Seitansicht dargestellten Stimulationselektrode 1 handelt es sich um eine unipolare Noppenelektrode mit einem einen zylinderförmigen Grundkörper 2 aus Titan aufweisenden Kopf. Der zylinderförmige Grundkörper 2 weist erfindungsgemäß eine aus einem inerten Material Iridiumnitrid (IrN) bestehende Oberflächenbeschichtung 3 auf, die mittels Kathodenzerstäubung auf den zylinderförmigen Grundkörpers 2 der Titanelektrode aufgebracht ist. Die Elektrode weist eine gewendelte, elektrisch leitende Zuleitung 4 auf, die mit einer elektrisch isolierenden Ummantelung 5 aus Silikon versehen ist. In der Zeichung ist diese Silikonummantelung transparent wiedergegeben. An die Silikonummantelung angeformt sind nach rückwärts gerichtete flexible Befestigungselemente 6 und 7, welche zur Verankerung der Elektrode im Herzen dienen, wobei die Oberfläche des Grundkörpers in Kontakt mit der inneren Herzoberfläche gehalten wird.

Der Grundkörper 2 ist mittels eines hohlzylindrischen Ansatzes 8 über die Zuleitung 4 geschoben und dort befestigt, wobei dieser Ansatz in der Zeichnung geschnitten dargestellt ist.

In Figur 2 ist ein Ausschnitt (Detail II in Figur 1) der aktiven Oberfläche vergrößerten wiedergegeben. Wie aus der Darstellung ersichtlich ist, wird durch die (unmaßstäblich vergrößerte) fraktale räumliche Geometrie der im mikroskopischen Bereich stengelartig gewachsenen Beschichtung 3 eine wesentliche Vergrößerung der aktiven Oberfläche erzielt. Die erzielte Oberflächenvergrößerung liegt im Bereich von mehr als 1000.

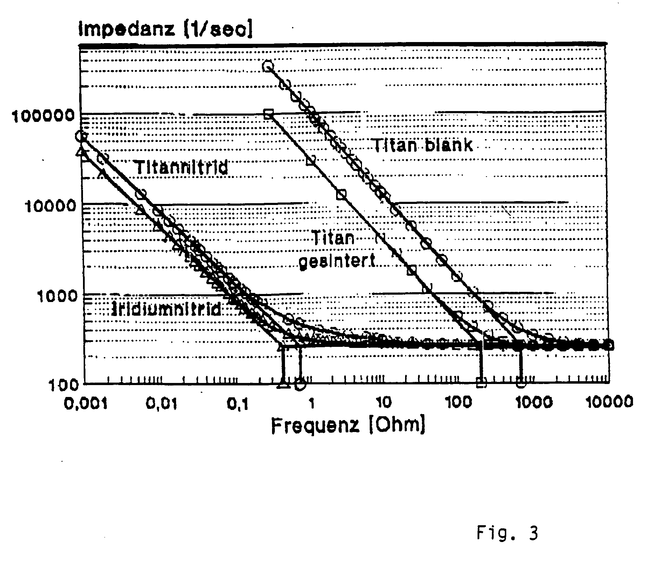

Aus Figur 3, die den Verlauf der Impedanzen von Stimulationselektroden mit unterschiedlichen Oberflächenbeschichtungen im Vergleich zeigt, ist ersichtlich, daß eine mit Iridiumnitrid beschichtete Elektrode insbesondere im Bereich kleiner, für den Empfang von aus dem Herzen aufnehmenden Signalen besonders wichtigen niederfrequenten Bereich im Vergleich zu den aus dem Stand der Technik bekannten Elektrodenoberflächenmaterialien Titan bzw. Titannitrid die niedrigste Phasengrenzimpendanz besitzt. Die ermittelten Unterschiede sind in ihren Auswirkungen deshalb besonders wesentlich, da die Amplitude des aufgenommenen Signals quadratisch mit dem Innenwiderstand der Signalquelle zusammenhängt.

Andere Ausführungsformen, von Schrittmacherelektroden, bei denen ein anodischer Betrieb betriebsmäßig gewollt ist, sind in den Zeichnungen nicht näher dargestellt. Sie zeichnen sich aus durch eine gegenüber vergleichbaren bekannten Elektroden verkleinerte Oberfläche, da auch hier auf gewisse Flächenreserven verzichtet werden kann, welche bei den bekannten Elektroden für den Fall der Impedanzvergößerung im Betrieb vorgesehen sein mußten. Bei bi- oder multipolaren Elektroden sind im gegenüber dem Elektrodenkopf zurückliegenden Teil ringförmige Bereiche vorgesehen, die mit separaten galvanischen Verbindungen zum anschlußseitigten Ende versehen sind. Hiermit kann dann entweder eine bipolare Stimulation oder aber eine intrakardiale Impedanzumessung zur Erfassung der Herzaktivität erfolgen.

Im Falle der Verwendung des Herzschrittmachergehäuses ist ein in Richtung zur Körperoberfläche gelegener Bereich des Gehäuses mit der erfindungsgemäßen Beschichtung versehen, während der übrige Teil des Gehäuses mit einer isolierenden Ummantelung versehen ist, die bevorzugt aus Silikon-Kautschuk besteht.

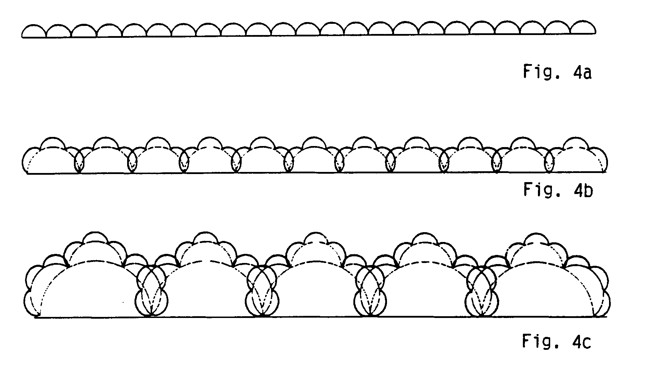

Aus der Darstellung in den Figur 4a bis c ist ersichtlich, wie die in Figur 4a dargestellte Grundform eines halbkreisförmigen Querschnittes überlagert wird von einer entsprechenden maßstäblich verkleinerten geometrischen Form. Die verkleinerten Formelemente lagern sich dabei jeweils an der Oberfläche der nächst größeren Grundform an. Die nächste Stufe der Überlagerungen ist dabei in Figur 4c wiedergegeben. Die vereinfachte Darstellung in diesen Figuren dient lediglich der Veranschaulichung der grundsätzlichen geometrischen Verhältnisse. Bei der praktischen Herstellung können sich die Grundformen räumlich weiteren Stufen überlagern.

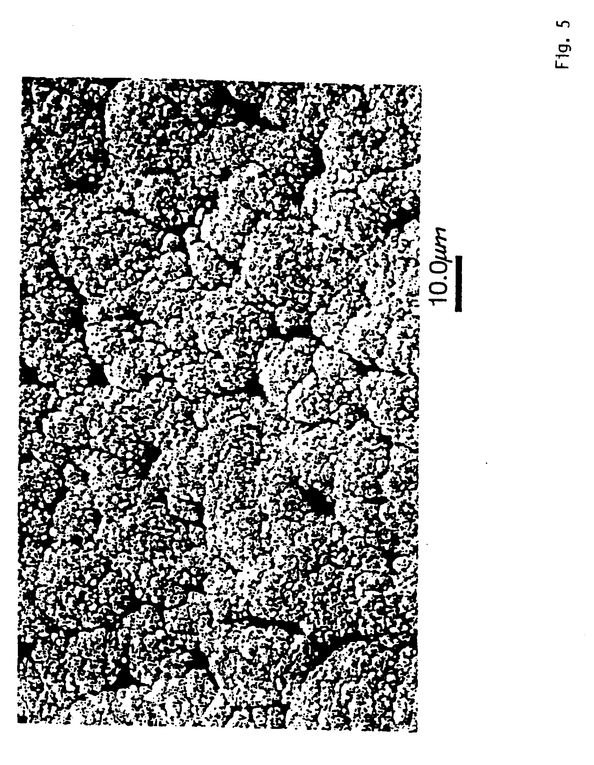

Die elektronenmikroskopisch vergrößerte Darstellung gemäß Figur 5 zeigt die Oberfläche einer erfindungsgemäßen Elektrode, die ein blumenkohlartiges Äußeres zeigt. Die Struktur ist zwar unregelmäßig geformt, folgt aber den dargestellten fraktalen Gesetzmäßigkeiten. Durch die sich nach außen hin stets verfeinernde Struktur ist eine mikroskopische Oberfläche erzielbar, die flächenmäßig um ein Vielfaches größer ist als der zugehörige makroskopische Flächenbereich.

Die Erfindung beschränkt sich in ihrer Ausführung nicht auf das vorstehend angegebene bevorzugte Ausführungsbeispiel.

## Patentansprüche

1. Stimulationselektrode mit einem Grundkörper und einer porösen Oberflächenbeschichtung deren aktive Oberfläche wesentlich größer ist als die sich aus der geometrischen Grundform der Elektrode ergebende Oberfläche, wobei die Oberflächenbeschichtung aus einem inerten Material, d.h. einem Material ohne bzw. mit einer nur sehr geringen Oxidationsneigung besteht, derart, daß das Material der Oberflächenbeschichtung aus einem inerten Element, einer inerten chemischen Verbindung und/oder einer inerten Legierung gebildet ist, **dadurch gekennzeichnet,** daß die aktive Oberfläche durch eine fraktalartige räumliche Geometrie um einen Faktor von mindestens tausend größer ist als die sich aus der geometrischen Grundform der Elektrode ergebende Oberfläche.

2. Stimulationselektrode nach Anspruch 1, **dadurch gekennzeichnet,** daß als inertes Material ein Nitrid, Carbid oder Carbonnitrid <u>der Elemente</u> oder ein reines Element bzw. eine Legierung aus der Gruppe Gold, Iridium, Platin oder Kohlenstoff vorgesehen ist.

3. Stimulationselektrode nach Anspruch 2, daß die Beschichtung aus Iridiumnitrid besteht.

4. Stimulationselektrode nach einem der vorangehenden Ansprüche, **dadurch gekennzeichnet,** daß die Oberflächenbeschichtung mittels Dünnschichttechnologie auf die Elektrode aufgebracht ist.

5. Stimulationselektrode nach Anspruch 4, **dadurch gekennzeichnet,** daß die Oberflächenbeschichtung mittels reaktiver Kathodenzerstäubung oder Ionenplattierung auf die Elektrode aufgebracht ist.

6. Stimulationselektrode nach einem der vorangehenden Ansprüche, **dadurch gekennzeichnet,** daß der Grundkörper aus Titan besteht.

## Claims

1. Stimulating electrode with a base body and a porous surface coating, the active surface of which is substantially greater than the surface resulting from the basic geometric shape of- the electrode, the surface coating consisting of an inert material, i.e. a material without any or with only a very slight tendency to oxidation, so that the material of the surface coating is formed from an inert element, an inert chemical compound and/or an inert alloy, characterised in that, due to a fractal-like three-dimensional geometry, the active surface is greater by a factor of at least a thousand than the surface resulting from the basic geometric shape of the electrode.

2. Stimulating electrode according to claim 1, characterised in that a nitride, carbide or carbon nitride of the elements or a pure element or an alloy from the gold, iridium, platinum or carbon group is provided as the inert material.

3. Stimulating electrode according to claim 2, characterised in that the coating consists of iridium nitride.

4. Stimulating electrode according to any one of the preceding claims, characterised in that the surface coating is applied to the electrode by means of thin-film technology.

5. Stimulating electrode according to claim 4, characterised in that the surface coating is applied to the electrode by means of reactive cathode sputtering or ion plating.

6. Stimulating electrode according to any one of the preceding claims, characterised in that the base body consists of titanium.

**Revendications**

1. Electrode de stimulation comprenant un corps de base et un revêtement de surface poreux dont la surface active est beaucoup plus grande que la surface résultant de la forme géométrique fondamentale de l'électrode, le revêtement de surface étant fait d'un matériau inerte, c'est-à-dire d'un matériau n'ayant pas de tendance à l'oxydation ou ayant seulement une très faible tendance à l'oxydation, le matériau du revêtement de surface étant constitué en particulier d'un élément inerte, d'un composé chimique inerte et/ou d'un alliage inerte, caractérisée en ce que la surface active, grâce à une géométrie spatiale de type fractal, est plus grande d'un facteur d'au moins mille que la surface résultant de la forme géométrique fondamentale de l'électrode.

2. Electrode de stimulation selon la revendication 1, caractérisée en ce que le matériau inerte est un nitrure, un carbure ou un carbonitrure des éléments, un élément pur ou un alliage du groupe comprenant l'or, l'iridium, le platine ou le carbone.

3. Electrode de stimulation selon la revendication 2, caractérisée en ce que le revêtement est en nitrure d'iridium.

4. Electrode de stimulation selon une des revendications précédentes, caractérisée en ce que le revêtement de surface est appliqué sur l'électrode selon la technologie des couches minces.

5. Electrode de stimulation selon la revendication 4, caractérisée en ce que le revêtement de surface est appliqué sur l'électrode par pulvérisation cathodique réactive ou par placage ionique.

6. Electrode de stimulation selon une des revendications précédentes, caractérisée en ce que le corps de base est en titane.

Fig. 1

Fig. 2

EP 0 597 995 B1

Fig. 3

8

Fig. 4a

Fig. 4b

Fig. 4c

Fig. 5

10.0μm